**Europäisches Patentamt**

**European Patent Office**

**Office européen des brevets**

(19)

(11) Publication number : **0 363 106 B1**

# (12) EUROPEAN PATENT SPECIFICATION

(45) Date of publication of patent specification :
**31.05.95 Bulletin 95/22**

(51) Int. Cl.⁶ : **G01N 33/569,** G01N 33/543, C07K 1/14

(21) Application number : **89310005.7**

(22) Date of filing : **29.09.89**

(54) Use of cationic surfactant to extract the chlamydial major outer membrane protein antigen.

(30) Priority : **07.10.88 US 255926**

(43) Date of publication of application :
**11.04.90 Bulletin 90/15**

(45) Publication of the grant of the patent :
**31.05.95 Bulletin 95/22**

(84) Designated Contracting States :
**CH DE FR GB IT LI SE**

(56) References cited :
**EP-A- 0 272 044**
**EP-A- 0 280 558**
**EP-A- 0 291 479**
**EP-A- 0 325 045**
**WO-A-87/02678**
**US-A- 4 302 549**
**US-A- 4 497 899**
**US-A- 4 746 614**

(73) Proprietor : **EASTMAN KODAK COMPANY (a New Jersey corporation)**
**343 State Street**
**Rochester, New York 14650 (US)**

(72) Inventor : **Mauck, John Charles, c/o Eastman Kodak Co.**
**Patent Dept.,**
**343 State Street**
**Rochester, NY 14650 (US)**
Inventor : **Green, Nancy Fame, c/o Eastman Kodak Co.**
**Patent Dept.,**
**343 State Street**
**Rochester, NY 14650 (US)**

(74) Representative : **Mercer, Christopher Paul et al**
**Carpmaels & Ransford**
**43, Bloomsbury Square**
**London WC1A 2RA (GB)**

## Description

The present invention relates to the extraction of the major outer membrane protein from chlamydial organisms. It also relates to a diagnostic assay for determining the presence of such organisms in a biological specimen using the extracted antigen.

Immunoassays have been used in recent years to detect the presence of infectious diseases. In order for the assay to be useful, it must detect a particular organism with a high degree of reliability. In most cases, this requires the isolation and reaction of antigens peculiar to the organism with corresponding antibodies. For the test to be commercially successful, it also needs to be relatively inexpensive, simple to use and rapid.

One such organism which can be detected by immunoassay is Chlamydial trachomatis (herein C. trachomatis) which is one of two microbial species of the genus Chlamydiaceae, order Chlamydiales. there are 15 or more strains of this species which are the causes of a number of human ocular and genital diseases including trachoma, inclusion conjunctivitis, lymphogranuloma venereum, nongonococcal urethritis and proctitis. Infection from C. trachomatis is pervasive in the general population so that it is believed that there are millions of cases each year of nongonococcal urethritis alone.

Because of the widespread nature of this disease, there is considerable interest in having a rapid, simple and reliable test for detection of chlamydial organisms. Considerable research has been carried out to find useful ways to extract detectable antigen from chlamydial organisms. For example, EP-A-0 325 045 describes the use of various anionic, cationic, nonionic and amphoteric detergents in antigen extraction procedures. Various detergents are mentioned for extraction of chlamydial antigens in US-A-4,497,899.

There are two chlamydial antigens which can be extracted from the organisms, the lipopolysaccharide and the major outer membrane protein (MOMP). The MOMP comprises 60% of the total associated outer membrane protein of C. trachomatis, and has a size or subunit molecular weight of between 38,000 and 44,000 daltons. Moreover, it is known that this antigen reacts with species specificity that is common to all serotypes. Thus, it can provide a basis for determination of all C. trachomatis serotypes.

US-A-4,427,782 describes the isolation of MOMP and its detection using standard analytical methods. The antigen is extracted from elementary bodies using a two step process: first mixing with a mild anionic detergent followed by mixing with a strong anionic detergent. Another extraction procedure using a detergent and reducing agent is described in EP-A-0 291 479. A simple and effective method for extracting MOMP would be highly desirable.

An improved method for extracting the major outer membrane protein of chlamydial organisms comprises:
A. providing a specimen suspected of containing chlamydial organisms, and
B. contacting the specimen with an extraction composition having a pH of at least 8 and comprising a sulfhydryl-containing reducing agent, and a cationic surfactant present in an amount of at least 0.1 mg/ml to extract chlamydial major outer membrane protein antigen from the organisms for detection, wherein the cationic surfactant is a quaternary ammonium salt of a polypropoxy-t-amine or a mixture thereof.

This invention also provides a method for the determination of chlamydial organisms comprising:
A. extracting chlamydial major outer membrane protein antigen from a specimen suspected of containing chlamydial organisms with an extraction composition having a pH of at least 8 and comprising a sulfhydryl-containing reducing agent, and a cationic surfactant present in an amount of at least 0.1 mg/ml to extract chlamydial major outer membrane protein antigen from the organisms for detection, wherein the cationic surfactant is a quaternary ammonium salt of a polypropoxy-t-amine or a mixture thereof,
B. contacting the extracted antigen with chlamydial antibodies to form an immunological complex, and
C. determining the presence of the complex as an indication of the presence of chlamydial organisms in the specimen.

The assay of this invention is rapid, reliable and simple to use. For example, it can be carried out in less than 30 minutes at room temperature. It is particular to the extraction and detection of the major outer membrane protein of chlamydial organisms (such as from C. trachomatis). The advantages noted herein are possible because the antigen is extracted using an extraction composition comprised of a sulfhydryl-containing reducing agent and a cationic surfactant present in an amount of at least 0.1 mg/ml. The cationic surfactant is a quaternary ammonium salt of a polypropoxy-t-amine or a mixture thereof. The extraction step itself is rapid, generally taking less than 10 minutes.

The present invention is a method for determining the presence of C. trachomatis (or other chlamydial species) in a biological specimen which has been obtained from a patient using any suitable medical or diagnostic techniques. Such specimens include, for example, swab specimens obtained from the cervix, urethra, throat or anus of a patient, and body fluids such as synovial fluid or fluid from lesions. The biological specimens so obtained are suspected of containing chlamydial organisms which comprise the chlamydial MOMP antigen to be determined.

The MOMP antigen is extracted from chlamydial organisms using a buffered composition containing one or more cationic surfactants. Generally such surfactants have one or more cationic groups selected from the group consisting of quaternary ammonium salts, quaternary phosphonium salts, quaternary pyridinium salts and quaternary imidazolium salts. Quaternary ammonium salts are preferred. Generally, any cationic surfactant is useful in the present invention as long as it does not adversely affect the bound antigen (once extracted) or the immunological complexes formed in the assay. Many cationic surfactants meeting those requirements are known in the art, and can be evaluated by routine experimentation. The standard resource of McCutcheon's Emulsifiers and Detergents, 1986 Edition, McCutcheon Division, Publishing Co., Glen Rock, N.J. 1986 can be consulted to find a number of useful cationic surfactants.

Useful cationic surfactants include, but are not limited to, nonpolymeric aliphatic, heterocyclic or carbocyclic compounds having a molecular weight less than 3000. Preferably, these compounds are aliphatic, heterocyclic or carbocyclic quaternary ammonium compounds.

As used herein, "aliphatic" refers to an organic cationic compound which contains aliphatic (or open-chain) groups connected to the atom (for example, a phosphorus or nitrogen) which provides the positive charge. These groups contain from 1 to 30 carbon atoms and can have oxygen or sulfur atoms interspaced along the chain, provided each compound has at least 1 carbon atom. One or more hydrogen atoms along any aliphatic chain can be replaced with fluorine atoms to provide a fluorinated group. The groups can also be substituted with one or more other halo atoms, aryl, alkoxy, amino, cycloalkyl or other groups as would be apparent to one skilled in the art.

As used herein, the term "heterocyclic" refers to an organic cationic compound having at least one heterocyclic moiety attached to the atom providing the cationic charge. The cationic charge can be within the heterocyclic group if desired, or in another portion of the molecule. It can be aromatic or nonaromatic and can contain nitrogen, sulfur, oxygen or selenium atoms as well as carbon atoms. Generally, the heterocyclic moiety has from 5 to 15 atoms in the backbone and can be substituted with one or more other organic groups if desired as would be apparent to one skilled in the art.

The term "carbocyclic" refers to an organic compound having one or more carbocyclic moieties attached to the atom providing the cationic charge. Such moieties include cycloalkyl generally of 5 to 20 carbon atoms, cycloalkenyls generally of 5 to 20 carbon atoms, and aryls generally of 6 to 14 carbon atoms, in the cyclic ring. They can be unsubstituted or substituted with one or more other organic groups as would be apparent to one skilled in the art.

Representative surfactants include polypropoxy quaternary ammonium chlorides, acetates and phosphates (marketed as Emcol™), fatty acid amidoalkyldimethyl amines (Schercodines, a tradename), ethoxylated fatty amines (Pegameens, a tradename), long-chain alkyldiethanol methyl quaternary ammonium chlorides (M-Quat™), fatty acid derivatives of imidazolines (Monazolines, a tradename), polyoxyethylene fatty amines (Mazeen™) and long-chain alkylhydroxyethyl imidazolines (Alkazines, a tradename). Most useful surfactants are the quaternary ammonium salts of polypropoxy-t-amines (Emcol™ CC-9, CC-36, CC-55 and CC-57 for example).

Others include, but are not limited to, nonyltrimethyl ammonium bromide, dodecyltrimethyl ammonium chloride, hexadecyltrimethyl ammonium bromide, hexadecylpyridinium bromide, benzyltriethyl ammonium chloride, didodecyldimethyl ammonium bromide, benzyldimethylphenyl ammonium chloride, tetrahexyl ammonium chloride, stearyldimethylbenzyl ammonium chloride, polypropoxy quaternary ammonium chlorides and perfluoroalkyl betaines (such as Fluorad FC 135, a tradename or Zonyl™ FSC). Mixtures of surfactants can be used if desired.

The amount of cationic surfactant in the solution is generally at least 0.1 mg/ml of the solution used to extract the antigen. Preferably, it is present in an amount of from 1 to 10 mg/ml. The amount may be adjusted for various surfactants to obtain the optimum results. A particularly useful extraction composition is described below in reference to the examples.

The extraction composition desirably has a pH of from 8 to 13 using any suitable buffer or strong base. A base can be used to adjust the pH and then the solution can be buffered with one or more buffers to maintain the pH. Alternatively, the buffer may be strong enough to adjust pH as well as to maintain it.

Examples include alkali metal, alkaline earth and ammonium hydroxides (such as sodium hydroxide, potassium hydroxide, calcium hydroxide and ammonium hydroxide), phosphates (such as sodium phosphate and potassium phosphate), tris(hydroxymethyl)aminomethane, 3-cyclohexylamino-1-propane sulfonic acid and related biological buffers. The amount of buffer in the extraction solution is chosen to provide suitable buffering capability. Mixtures of buffers can be used if desired.

In addition, the extraction composition can include one or more other reagents which facilitate lysis of the organism and extraction of the MOMP antigen. Examples of such reagents include sulfhydryl-containing reducing agents, including thiols such as 1,3-dimercapto-2-propanol, 2,3-dimercapto-1-propanol, 1,2-dimercaptoethane, dithiothreitol, dithioerythritol, mercaptoethanol and thioglycerol. Other useful reducing agents are glutathione, N-actylcysteine, cysteine, thioglycolic acid, L-cysteinemethyl ester, L-cysteineethyl ester and N-acetyl-D,L-isocysteine. The thiols are preferred with dithiothreitol being most preferred in the practice of this invention.

Other optional, but preferred addenda, include alcoholamines, such as ethanolamine, propanolamine, diethanolamine, triethanolamine and salts thereof.

Extraction is generally carried out at room temperature (that is, 18-25°C), although lower or higher temperatures can be used if desired. But the higher temperatures are not needed and may be undesirable with certain specimens.

Once antigen is extracted from the organism, it is desirable, although not essential, that the specimen be prefiltered to remove cell debris, particulate matter and other unwanted materials prior to further handling. Prefiltering can be carried out in a suitable container having a filter of some type.

Extraction can also be carried out in a suitable container, such as a test tube, beaker, cuvette or other device known to one skilled in the art. Particularly useful devices known in the art include those shown in U.S. Patent 4,746,614.

The filtered specimen is then subjected to any of a number of analytical procedures in order to determine the presence of extracted antigen. Such procedures include culture techniques, counter-immunoelectrophoresis and serological tests which, while not preferred, may be the only choice in certain instances.

Preferably, the extracted antigen is detected using an immunoassay in which it is immunologically reacted with one or more appropriate antibodies. The resulting immunological complex is detected using a suitable radiometric, colorimetric, fluorometric or enzyme labeled reagent. In some cases, the reagent is a labeled antibody to the antigen, and in other cases, the labeled anti-antibody is directed to an unlabeled antibody which is reactive with the antigen. Such immunoassays generally include the formation of a detectable immunological complex on a solid support of some type, either coated or uncoated, followed by appropriate detection procedures. Other assays involve agglutination of the immunological complex when at least one reactant (such as an antibody) of the complex is attached to labeled or unlabeled particles of some type that clump together during complex formation.

Examples of useful assays include competitive immunoassays or enzyme-linked immunoabsorbent assays (or what is commonly called "ELISA"). Such assays are described generally in U.S. Patent 4,427,782 and by Schmeer et al, J. Clin. Microbiol., 15(5), pp. 830-834 (1982).

Preferably, extracted MOMP antigen is contacted with a polymeric solid support to which it can become bound. Useful support materials include glass, cellulosic or polymeric beads, films, tubes, plates and others known in the art. Preferably, the support material is a microporous membrane as described in more detail below. This membrane can be "bare", that is uncoated or untreated with any substance (as shown in US-A-4,497,899). However, it may be treated or coated with a substance (such as a surfactant) which may enhance assay performance.

In certain embodiments, solid supports are used which have a multiplicity of positively charged groups on the surface thereof. These positively charged groups produce a positive zeta potential over a wide pH range. Zeta potential is known as the potential between the support and a fluid in contact with it. Any positively charged chemical radical which produces the desired zeta potential on the support is useful in the practice of this invention.

For example, the support can be constructed of any natural or synthetic polymeric material with suitable cationic groups thereon which will ionically bind to the extracted antigen. Useful charged polymers include polyesters, polyamides, polyethyleneimines, polycarbonates, cellulosic materials, addition polymers prepared from ethylenically unsaturated vinyl monomers and others known in the art having the requisite charged groups. Generally, the cationic groups are quaternary ammonium salts, quaternary phosphonium salts, quaternary sulfonium salts, quaternary pyridinium salts, quaternary pyrimidinium salts or quaternary imidazolium salts. Quaternary ammonium salts are preferred.

One useful polymeric solid support is a microporous membranes known as Posidyne™ or Biodyne™ B. These supports comprise a nylon membrane coated with a polyester which has quaternary ammonium groups in the pores. Other useful supports include polymeric membranes coated with surfactants.

In contrast to the embodiments described above wherein the antigen is bound to the solid support prior to immunological reaction, the assay of this invention can also be carried out by forming the immunological complex simultaneously with or prior to attachment to the solid support. In other words, the complex can be formed in solution followed by contact with the solid support for binding thereto.

The support described herein can be used in combination with other equipment (bottles, test tubes, swabs, beakers or cups) in order to carry out the assay. Alternatively and preferably, the support is a microporous membrane which is fitted into a disposable test device in which the assay can be carried out and all fluids accommodated. Useful configurations of test devices are known in the art including U.S. Patents 3,825,410, 3,888,629, 3,970,429 and 4,446,232. Particularly useful devices are described in EP-A-0 280 558 and EP-A-0 308 231.

Almost immediately upon contact of the antigen with the charged support, the antigen is bound to the support. If desired, any unbound antigen may be removed from the support by washing with any suitable wash solution, but particularly a wash solution comprising a cationic surfactant.

Within 10 minutes, and preferably within 1 to 5 minutes, of the contact, the bound antigen is contacted with chlamydial antibody so as to form an immunological complex on the support. Fluid and unbound materials can be removed quickly at the same time. If the assay is carried out using a disposable test device, the support can be a microporous membrane through which fluid and unbound materials in the specimen are filtered as the antigen is bound to the membrane.

The antibody used in this assay is specifically immunoreactive with the MOMP antigen of one or more chlamydial strains. It can be polyclonal or monoclonal. If polyclonal, it is commercially available or prepared in various animals using known techniques employing an antigen common to the strain of organism to be detected. A single antibody or mixture thereof can be used. Preferably, the antibodies are monoclonal which are either commercially available or prepared using standard hybridoma technology. Useful procedures for preparing antibodies are described, for example, in U.S. Patent 4,427,782.

In one embodiment, the antibody to the antigen is labeled for detection. Useful labels are known in the art and include chemical or biological compounds which are directly detectable using suitable procedures and equipment, as well as compounds which can be detected through further chemical or specific binding reactions to provide a detectable species. Examples of useful labels include radioisotopes, enzymes, fluorescent compounds, chemiluminescent compounds, phosphorescent compounds, biotin or its derivatives, avidin or its derivatives, ferritin, magnetizable particles, dyed particles and others readily apparent to one skilled in the art. Radioisotopes or enzymes are preferred labels. The labels can be attached to antibodies using known techniques. Where the label is not directly detectable, further reagents or compounds are needed to render the reaction or specific binding product detectable. For example, if the label is biotin, it can be reacted with avidin which is conjugated with an enzyme to provide a detectable species. Where the label is an enzyme, such as glucose oxidase, urease, peroxidase, alkaline phosphatase and others, substrates and dye-providing reagents are also needed. Alkaline phosphatase and peroxidase are particularly useful enzyme labels.

In a preferred embodiment, the label is peroxidase, and at some point in the assay, hydrogen peroxide and suitable dye-forming reagents are added to provide a detectable dye. For example, useful dye-providing reagents include leuco dyes, such as triarylimidazole leuco dyes (as described in U.S. Patents 4,089,747, or other compounds which react to provide a dye in the presence of peroxidase and hydrogen peroxide (that is, compounds which react to provide a dye upon catalytic action of peroxidase).

In a preferred embodiment, the chlamydial antibody is not labeled, and detection of the antibody-antigen complex formed and bound to the support is accomplished using a second antibody (described below) which is specific to the unlabeled antibody and is appropriately labeled.

The chlamydial antibody (labeled or unlabeled) can be contacted with the bound antigen in the presence of one or more proteins which reduce nonspecific interactions on the support. Useful proteins are well known and include, for example, casein, $\alpha$-casein, fetal bovine serum and porcine gamma globulin. A particularly useful blocking composition comprises a nonimmunological protein and an amphoteric surfactant.

Once the bound antigen has been contacted with the chlamydial antibody, a bound immunological complex is formed on the support. To hasten the formation of this complex, the antibody and antigen are generally incubated at a temperature of from 15 to 30°C for up to 10 minutes. Preferably, the incubation is carried out at from 18 to 25°C (that is, room temperature) for from 1 to 5 minutes. These mild incubation conditions are in sharp contrast to the 30 minutes at 37°C described as necessary for adsorption of chlamydial antigen to bare supports in U.S. Patent 4,497,899.

After the incubation and within 10 minutes of the antibody-antigen contact, the bound complex is washed one or more times with a suitable wash solution as described above. A particularly useful wash solution is described below in reference to the examples.

In the embodiment described above where the chlamydial antibody is labeled, the assay procedure after washing is to detect the label directly or indirectly after addition of the appropriate reagents. This is done relatively quickly after washing the bound complex, that is generally within 10 minutes, and preferably within 1 to 5 minutes. If desired, label detection can be hastened with incubation if the reagents warrant it. The label is then detected using standard equipment and procedures.

In a preferred embodiment, the chlamydial antibody is unlabeled, and after washing the bound complex, it is contacted with an antibody directed to the unlabeled antibody. This second antibody (that is, an anti-antibody) is appropriately labeled with any of the labels described above. The antibody can be monoclonal or polyclonal and either purchased or prepared using known techniques.

After this contact, the resulting labeled antigen-antibody-antibody complex which is bound to the support is incubated for up to 10 minutes at a temperature of from 15 to 30°C, and preferably for 1 to 5 minutes at from 18 to 25°C.

Further washing can be carried out using other suitable wash solution to remove uncomplexed materials, and suitable enzyme substrates or other needed reagents are added to provide a detectable species. The bound labeled antigen-antibody-labeled antibody complex is then detected on the support using standard radiometric, colorimetric, fluorescent or other detection techniques.

A preferred method for the determination of chlamydial organisms comprising:

A. extracting chlamydial major outer membrane protein antigen from a specimen suspected of containing chlamydial organisms with an extraction composition having a pH of at least 8 comprising a sulfhydryl-containing reducing agent and a cationic surfactant present in an amount of at least 0.1 mg/ml, wherein the cationic surfactant is a quaternary ammonium salt of a polypropoxy-t-amine or a mixture thereof,

B. contacting the extracted antigen with a solid support so as to bind the antigen to the support,

C. prior to, simultaneously with or subsequently to contacting step B, contacting the extracted antigen with unlabeled chlamydial antibodies to form an immunological complex,

D. washing unbound materials away from the bound complex on the support,

E. contacting the bound complex with labeled antibodies directed to the unlabeled chlamydial antibody to form a labeled antigen-antibody-antibody complex bound to the support, and

F. determining the presence of the labeled complex as an indication of the presence of chlamydial organisms in the specimen.

The following examples are provided to illustrate, but not limit the scope of, the present invention.

To perform these examples, a mouse monoclonal antibody to the chlamydial major outer membrane protein antigen (anti-MOMP) was prepared using standard hybridoma technology and mouse cell line and stored in a solution of phosphate buffered saline solution (pH 7.4) containing 0.01% (by weight) azide. The antibody composition used in the assay was prepared by adding a sample (19 µl) of the antibody solution to a phosphate buffered saline solution (diluting 1:800) containing casein (0.5 weight %) as a blocking protein and Lonzaine™ C amphoteric surfactant (0.01 weight %, available from Lonza, Inc.), then filtered through a 0.22 µmeter filter to obtain a working solution.

The labeled polyclonal antibody used was a goat anti-mouse IgG antibody conjugated to horseradish peroxidase which was obtained from BioRad Laboratories. These conjugate was diluted to 1:2000 in a phosphate buffered saline solution containing 0.5% (by weight) casein and 0.01% (by weight) Lonzaine™ C amphoteric surfactant, and filtered through a 0.22 µmeter filter to obtain a working solution.

An antigen extraction solution was prepared by dissolving the following components in water: sodium azide (15 mmolar), sodium chloride (0.15 mmolar), dithiothreitol reducing agent (7.5 mmolar), ethanolamine (0.26 mmolar), ethylenediaminetetraacetic acid (25 mmolar) and sodium hydroxide (0.1 molar, pH adjusted to 12.5). To 15 ml of this solution was added 375 µl of a 10% (by weight) solution of Emcol™ CC-36 cationic surfactant (quaternary ammonium chlorides of polypropoxy-t-amines) in methanol.

Example 1: Extraction of Major Outer Membrane Protein from Chlamydial Organisms

This example demonstrates the practice of the present invention to extract C. trachomatis major outer membrane protein antigen.

Elementary body protein (81.5 µl in bovine serum albumin/phosphate buffered saline solution, 1500 pg final concentration) was added to two extraction compositions (each 1418.5 µl), one being that described above containing Emcol™ CC-36 cationic surfactant, and the second being a Control composition lacking the surfactant, and mixed and held at room temperature for about 5 minutes. Two corresponding solutions were prepared without antigen.

Hydrogen peroxide solution (8 weight %, 1500 µl) was added to each extraction solution to remove endogenous catalase, peroxidase and myeloperoxidase. The resulting mixtures were again held at room temperature for 5 minutes.

A portion of each extraction solution (120 µl) was added to the wells of an individual disposable test device, and fluid was allowed to flow through immediately. The device contained a 5 µm microporous membrane having quaternary ammonium groups on the surface thereof commercially available as Biodyne™ B membrane. Prior to use, the membrane was treated with Zonyl™ FSN (a nonionic fluorinated surfactant).

A portion (120 µl) of the anti-MOMP solution (described above) was added to each well of the test device while fluid was allowed to flow through. Incubation was carried out at room temperature for about 5 minutes. Following incubation, the resulting antigen-antibody complex was washed twice with a buffered solution (pH 7.2) of phosphate buffer and Emcol™ CC-9 cationic surfactant (0.75 weight %, similar to Emcol™ CC-36 described above).

A solution (120 µl) of the goat anti-mouse antibodies labeled with peroxidase was added to each well and allowed to flow through the membrane upon contact. Incubation at room temperature was carried out again for 5 minutes to form an antigen-antibody-labeled antibody complex ionically bound to the membrane.

After washing twice with the wash solution described above, a dye-providing composition was added to each test well. This composition included hydrogen peroxide (10 mmolar), 2-(4-hydroxy-3-methoxyphenyl)-4,5-bis(4-methoxyphenyl)imidazole leuco dye (0.005 weight %), poly(vinyl pyrrolidone) (1 weight %), 4'-hydroxyacetanilide (5 mmolar) and diethylene triaminepentaacetic acid (10 mmolar).

Within about 10 minutes, a red dye was observed in the test well indicating the presence of chlamydial MOMP antigen obtained from the specimen. The transmission densities were measured and the results are provided in Table I below as the density difference ($\Delta D_T$) between extraction solutions with cationic surfactant and those without it. They indicate that this method was effective in extracting the MOMP antigen from the chlamydial organisms.

### TABLE I

#### $\Delta D_T$ for Extraction Compositions

| Antigen | Control | Invention |
|---------|---------|-----------|
| MOMP | 0.076 | 0.179 |

## Claims

1. A method for extracting major outer membrane protein antigen from chlamydial organisms comprising:
   A. providing a specimen suspected of containing chlamydial organisms, and
   B. contacting the specimen with an extraction composition having a pH of at least 8 and comprising a sulfhydryl-containing reducing agent, and a cationic surfactant present in an amount of at least 0.1 mg/ml to extract chlamydial major outer membrane protein antigen from the organisms for detection, wherein the cationic surfactant is a quaternary ammonium salt of a polypropoxy-t-amine or a mixture thereof.

2. The method as claimed in claim 1 wherein the contacting is carried out at room temperature for up to 10 minutes.

3. The method as claimed in either of claims 1 and 2 carried out in an extraction device.

4. A method for the determination of chlamydial organisms comprising:
   A. extracting chlamydial major outer membrane protein antigen from a specimen suspected of containing chlamydial organisms with an extraction composition having a pH of at least 8 and comprising a sulfhydryl-containing reducing agent, and a cationic surfactant present in an amount of at least 0.1 mg/ml to extract chlamydial major outer membrane protein antigen from the organisms for detection, wherein the cationic surfactant is a quaternary ammonium salt of a polypropoxy-t-amine or a mixture thereof,
   B. contacting the extracted antigen with chlamydial antibodies to form an immunological complex, and
   C. determining the presence of the complex as an indication of the presence of chlamydial organisms in the specimen.

5. The method as claimed in claim 4 wherein the chlamydial antibodies are labeled for detection.

6. The method as claimed in claim 5 wherein the chlamydial antibodies are labeled with an enzyme.

7. The method as claimed in claim 4 wherein the chlamydial antibodies are unlabeled, and the antibody-antigen complex is contacted with antibodies directed to the chlamydial antibodies, the anti-antibodies being labeled for detection.

8. The method as claimed in claim 7 wherein the anti-antibodies are labeled with an enzyme.

7

**9.** The method as claimed in any of claims 4 to 8 wherein the extracted antigen is bound to a solid support prior to contact with the chlamydial antibodies.

**10.** The method as claimed in claim 9 wherein the solid support is a microporous membrane in a disposable test device.

**11.** A method for the determination of chlamydial organisms comprising:
A. extracting chlamydial major outer membrane protein antigen from a specimen suspected of containing chlamydial organisms with an extraction composition having a pH of at least 8 and comprising a sulfhydryl-containing reducing agent, and a cationic surfactant present in an amount of at least 0.1 mg/ml to extract chlamydial major outer membrane protein antigen from the organisms for detection, wherein the cationic surfactant is a quaternary ammonium salt of a polypropoxy-t-amine or a mixture thereof,
B. contacting the extracted antigen with a solid support so as to bind the antigen to the support,
C. prior to, simultaneously with or subsequently to contacting step B, contacting the extracted antigen with unlabeled chlamydial antibodies to form an immunological complex,
D. washing unbound materials away from the bound complex on the support,
E. contacting the bound complex with labeled antibodies directed to the unlabeled chlamydial antibodies to form a labeled antigen-antibody-antibody complex bound to the support, and
F. determining the presence of the labeled complex as an indication of the presence of chlamydial organisms in the specimen.

**12.** The use of a composition to extract chlamydial major outer membrane protein antigen having a pH of at least 8 and comprising a sulfhydryl-containing reducing agent, and a cationic surfactant present in an amount of at least 0.1 mg/ml, the cationic surfactant being a quaternary ammonium salt of a polypropoxy-t-amine or a mixture thereof.

**Patentansprüche**

**1.** Verfahren zur Extraktion von Hauptprotein-Antigen der äußeren Membran von chlamydialen Organismen, bei dem man:
A. eine Probe bereitstellt, von der angenommen wird, daß sie chlamydiale Organismen enthält, und bei dem man
B. die Probe mit einer Extraktions-Zusammensetzung in Kontakt bringt, die einen pH-Wert von weniger als 8 aufweist und ein eine Sulfhydrylgruppe enthaltendes Reduktionsmittel enthält sowie ein kationisches oberflächenaktives Mittel in einer Menge von mindestens 0,1 mg/ml, um das chlamydiale Hauptprotein-Antigen der äußeren Membran von dem Organismus für eine Bestimmung zu extrahieren, wobei das kationische oberflächenaktive Mittel ein quaternäres Ammoniumsalz eines Polypropoxy-t-amins oder einer Mischung hiervon ist.

**2.** Verfahren nach Anspruch 1, bei dem das Kontaktieren bei Raumtemperatur über einen Zeitraum bis zu 10 Minuten durchgeführt wird.

**3.** Verfahren nach einem der Ansprüche 1 und 2, das in einer Extraktionsvorrichtung durchgeführt wird.

**4.** Verfahren zur Bestimmung von chlamydialen Organismen, bei dem man:
A. chlamydiales äußeres Hauptmembranprotein-Antigen von einer Probe extrahiert, von der angenommen wird, daß sie chlamydiale Organismen enthält, mit einer Extraktionszusammensetzung, die einen pH-Wert von mindestens 8 aufweist und ein eine Sulfhydrylgruppe enthaltendes Reduktionsmittel enthält sowie ein kationisches oberflächenaktives Mittel in einer Menge von mindestens 0,1 mg/ml, um chlamydiales Hauptprotein-Antigen der äußeren Membran des Organismus zur Bestimmung zu extrahieren, wobei das kationische oberflächenaktive Mittel ein quaternäres Ammoniumsalz eines Polypropoxy-t-amins oder einer Mischung hiervon ist, bei dem man
B. das extrahierte Antigen mit chlamydialen Antikörpern unter Bildung eines immunologischen Komplexes in Kontakt bringt, und bei dem man
C. das Vorhandensein des Komplexes bestimmt, als Anzeichen für das Vorhandensein von chlamydialen Organismen in der Probe.

5. Verfahren nach Anspruch 4, bei dem die chlamydialen Antikörper für die Bestimmung markiert sind.

6. Verfahren nach Anspruch 5, bei dem die chlamydialen Antikörper mit einem Enzym markiert sind.

7. Verfahren nach Anspruch 4, bei dem die chlamydialen Antikörper nicht markiert sind, und bei dem der Antikörper-Antigenkomplex mit Antikörpern in Kontakt gebracht wird, die auf die chlamydialen Antikörper gerichtet sind, wobei die Anti-Antikörper für die Bestimmung markiert sind.

8. Verfahren nach Anspruch 7, bei dem die Anti-Antikörper mit einem Enzym markiert sind.

9. Verfahren nach einem der Ansprüche 4 bis 8, bei dem das extrahierte Antigen an einen festen Träger vor dem Kontakt mit den chlamydialen Antikörpern gebunden wird.

10. Verfahren nach Anspruch 9, bei dem der feste Träger eine mikroporöse Membran in einer Wegwerf-Testvorrichtung ist.

11. Verfahren zur Bestimmung von chlamydialen Organismen, bei dem man:
A. von einer Probe, von der erwartet wird, daß sie chlamydiale Organismen enthält, chlamydiales Hauptprotein-Antigen der äußeren Membran extrahiert, und zwar mit einer Extraktions-Zusammensetzung mit einem pH-Wert von mindestens 8 und mit einem Gehalt an einem eine Sulfhydrylgruppe aufweisenden Reduktionsmittel sowie mit einem Gehalt an einem kationischen oberflächenaktiven Mittel in einer Menge von mindestens 0,1 mg/ml, um chlamydiales Hauptprotein-Antigen der äußeren Membran von dem Organismus zum Zwecke der Bestimmung zu extrahieren, wobei das kationische oberflächenaktive Mittel ein quaternäres Ammoniumsalz eines Polypropoxy-t-amins oder eine Mischung hiervon ist, bei dem man
B. das extrahierte Antigen mit einem festen Träger in Kontakt bringt, um das Antigen an den Träger zu binden, bei dem man
C. vor, gleichzeitig mit oder nach der Kontaktstufe B das extrahierte Antigen mit nicht markierten chlamydialen Antikörpern in Kontakt bringt, um einen immunologischen Komplex zu erzeugen, bei dem man
D. nicht gebundene Materialien von dem gebundenen Komplex auf dem Träger wegwäscht, bei dem man
E. den gebundenen Komplex mit markierten Antikörpern in Kontakt bringt, die auf die nicht markierten chlamydialen Antikörper gerichtet sind, um einen markierten Antigen-Antikörper-Antikörperkomplex zu bilden, der an den Träger gebunden ist, und bei dem man
F. das Vorhandensein des markierten Komplexes als Anzeichen für das Vorhandensein von chlamydialen Organismen in der Probe bestimmt.

12. Verwendung einer Zusammensetzung zur Extraktion von chlamydialem äußerem Hauptmembran-Protein-Antigen mit einem pH-Wert von mindestens 8 und mit einem Gehalt an einem eine Sulfhydrylgruppe aufweisenden Reduktionsmittel sowie einem kationischen oberflächenaktiven Mittel, das in einer Menge von mindestens 0,1 mg/ml vorliegt, wobei das kationische oberflächenaktive Mittel ein quaternäres Ammoniumsalz eines Polypropoxy-t-amins oder einer Mischung hiervon ist.

## Revendications

1. Procédé pour extraire l'antigène protéinique principal provenant de la membrane externe d'organismes chlamydiaux, comprenant :
A. la fourniture d'un échantillon supposé contenir des organismes chlamydiaux, et
B. la mise en contact de l'échantillon avec une composition d'extraction ayant un pH d'au moins 8 et comprenant un agent réducteur sulfhydrylé et un tensioactif cationique présent en une quantité d'au moins 0,1 mg/ml pour extraire l'antigène protéinique principal de la membrane externe chlamydiale des organismes pour la détection, dans lequel le tensioactif cationique est un sel d'ammonium quaternaire d'une polypropoxy-t-amine ou un mélange de tels sels.

2. Procédé selon la revendication 1, dans lequel la mise en contact est accomplie à la température ambiante pendant jusqu'à 10 min.

**3.** Procédé selon l'une quelconque des revendications 1 et 2, mis en oeuvre dans un dispositif d'extraction.

**4.** Procédé pour la détermination d'organismes chlamydiaux, comprenant :

A. l'extraction de l'antigène protéinique principal de la membrane externe chlamydiale d'un échantillon supposé contenir des organismes chlamydiaux avec une composition d'extraction ayant un pH d'au moins 8 et comprenant un agent réducteur sulfhydrylé et un tensioactif cationique présent en une quantité d'au moins 0,1 mg/ml pour extraire l'antigène protéinique principal de la membrane externe chlamydiale des organismes pour la détection, dans lequel le tensioactif cationique est un sel d'ammonium quaternaire d'une polypropoxy-t-amine ou un mélange de tels sels,

B. la mise en contact de l'antigène extrait avec des anticorps chlamydiaux pour former un complexe immun, et

C. la détermination de la présence du complexe en tant qu'indication de la présence d'organismes chlamydiaux dans l'échantillon.

**5.** Procédé selon la revendication 4, dans lequel les anticorps chlamydiaux sont marqués pour la détection.

**6.** Procédé selon la revendication 5, dans lequel les anticorps chlamydiaux sont marqués avec une enzyme.

**7.** Procédé selon la revendication 4, dans lequel les anticorps chlamydiaux ne sont pas marqués et le complexe anticorps-antigène est mis en contact avec des anticorps dirigés contre les anticorps chlamydiaux, les anti-anticorps étant marqués pour la détection.

**8.** Procédé selon la revendication 7, dans lequel les anti-anticorps sont marqués avec une enzyme.

**9.** Procédé selon l'une quelconque des revendications 4 à 8, dans lequel l'antigène extrait est lié à un support solide avant le contact avec les anticorps chlamydiaux.

**10.** Procédé selon la revendication 9, dans lequel le support solide est une membrane microporeuse dans un dispositif de test jetable.

**11.** Procédé pour la détermination d'organismes chlamydiaux, comprenant :

A. l'extraction de l'antigène protéinique principal de la membrane externe chlamydiale d'un échantillon supposé contenir des organismes chlamydiaux avec une composition d'extraction ayant un pH d'au moins 8 et comprenant un agent réducteur sulfhydrylé et un tensioactif cationique présent en une quantité d'au moins 0,1 mg/ml pour extraire l'antigène protéinique principal de la membrane externe chlamydiale des organismes pour la détection, dans lequel le tensioactif cationique est un sel d'ammonium quaternaire d'une polypropoxy-t-amine ou un mélange de tels sels,

B. la mise en contact de l'antigène extrait avec un support solide de manière à lier l'antigène au support,

C. avant, pendant ou après l'étape de mise en contact B, la mise en contact de l'antigène extrait avec des anticorps chlamydiaux non marqués pour former un complexe immun,

D. la séparation des substances non liées d'avec le complexe lié sur le support par lavage,

E. la mise en contact du complexe lié avec des anticorps marqués dirigés contre les anticorps chlamydiaux non marqués pour former un complexe antigène-anticorps-anticorps marqué lié au support, et

F. la détermination de la présence du complexe marqué en tant qu'indication de la présence d'organismes chlamydiaux dans l'échantillon.

**12.** Utilisation d'une composition pour extraire l'antigène protéinique principal de la membrane externe chlamydiale ayant un pH d'au moins 8 et comprenant un agent réducteur sulfhydrylé et un tensioactif cationique présent en une quantité d'au moins 0,1 mg/ml, le tensioactif cationique étant un sel d'ammonium quaternaire d'une polypropoxy-t-amine ou un mélange de tels sels.